# EUROPEAN PATENT SPECIFICATION

(11) **EP 2 712 613 B1**
(45) Date of publication and mention of the grant of the patent: **08.02.2017**
(21) Application number: 12186617.2
(22) Date of filing: 28.09.2012
(51) Int. Cl.: A61K 31/122, A61P 17/00, A61P 17/02

(54) **Vitamin K1 and uses thereof**
Vitamin K1 und Verwendungen davon
Vitamine K1 et ses utilisations

(43) Date of publication of application: 02.04.2014
(73) Proprietor: Pharmadab d.o.o., 1000 Ljubljana (SI)
(72) Inventor: Babnik, Anastazija, 1000 Ljubljana (SI)
(74) Representative: Engelhard, Markus

(56) References cited:
- WO-A1-99/63982
- WO-A1-2007/138103
- WO-A2-2006/113479
- CHU D ET AL: "Risk of hand-foot skin reaction with the multitargeted kinase inhibitor sunitinib in patients with renal cell and non-renal cell carcinoma: A meta-analysis", CLINICAL GENITOURINARY CANCER 2009 CANCER INFORMATION GROUP, LP USA, vol. 7, no. 1, 2009, pages 11-19, XP002689460, ISSN: 1558-7673

## Description

### Technical field of the invention

The present invention relates to vitamin K1 and to pharmaceutical formulations comprising vitamin K1 for use in methods of treatment involving the administration of vitamin K1 or of pharmaceutical formulation comprising vitamin K1.

### Background of the invention

Vascular endothelial growth factor (VEGF) is a signal protein produced by cells that stimulates vasculogenesis and angiogenesis. It is part of the system that restores the oxygen supply to tissues when blood circulation is inadequate.

VEGF's normal function is to create new blood vessels during embryonic development, new blood vessels after injury, muscle following exercise, and new vessels (collateral circulation) to bypass blocked vessels. When VEGF is over expressed, it can contribute to disease. Solid cancers cannot grow beyond a limited size without an adequate blood supply and cancers that can express VEGF are able to grow and metastasize. Overexpression of VEGF can cause vascular disease in the retina of the eye and other parts of the body.

Drugs such as bevacizumab or sorafenib or sunitinib can inhibit VEGF and control or slow those diseases. Anti-angiogenesis inhibitors target the blood vessels that supply oxygen to the cells, ultimately causing the cells to starve. Anti-angiogenic therapies are improving outcomes for many cancer patients, in many cases dramatically. While these therapies are generally well tolerated, they are still associated with a variety of distinctive side effects including rash, skin discoloration, dry skin, alopecia, and hair and nail changes.

Skin rash associated with sorafenib closely resembles seborrheic dermatitis, and appears as a homogeneous erythematous and squamous eruption across the mid-facial area and scalp. In a prospective, placebo-controlled study of 85 metastatic renal cell carcinoma (mRCC) patients, facial eruption occurred in 63% of sorafenib treated patients compared with 2% in the placebo group 32 (Autier J., etc., Arch Dermatol, 2008). Rash typically developed within the first 1-2 weeks of treatment.

Chemotherapy-induced acral erythema (also refers to "palmar-plantar erythrodysesthesia'" or "Hand Foot Skin Reaction" or 'Hand Foot Syndrome (HFS)") is reddening, swelling, numbness and desquamation on palms and soles that can occur after chemotherapy in patients with cancer. The cellular defects underlying Hand Foot Syndrome likely involve damage to the dermal capillary endothelium, which could increase susceptibility of hand and foot surfaces to mechanical injury and stress after subclinical trauma.

Several prophylactic measures have been suggested to prevent or reduce the severity of HFS and skin rash, which includes non-pharmacologic and pharmacologic treatments. Non-pharmacologic treatments such as moisturizing creams and aloe vera lotion have been disclosed for treating HFS induced by chemotherapy including anti-VEGF therapy (Chu et al., 2008, Lacouture et al., 2008). US 6979688 discloses using uracil ointment to prevent HFS which occurs during the systemic administration of 5-fluorouracil as cancer treatment. EP 2368549 discloses a therapeutic application of Clonidine or its derivatives in the treatment of HFS associated with anti-VEGF therapy. The aforementioned measures are either unspecific (non-pharmacologic treatments), or are useful only in the context of 5-fluorouracil chemotherapy, or they may have unwanted side effects, such as dizziness, hypotension and dry mouth.

Accordingly, an object of the present invention is to provide methods and means for an effective and economical treatment and prevention of skin reactions secondary to an anti-VEGF therapy.

### Summary of the invention

The object of the present invention is solved by Vitamin K1 for use in a method for treating or preventing a skin reaction associated with anti-VEGF therapy such as skin rash or Hand Foot Syndrome (HFS).

The object of the present invention also provides Vitamin K1 for use in a method for treating or preventing skin reactions associated with anti-VEGF therapy, such as skin rash or HFS which method comprises administering Vitamin K1 to a patient, preferably a human patient.

The object of the present invention is further solved by providing Vitamin K1 for use in the manufacture of a pharmaceutical formulation for treating or preventing skin reactions such as skin rash and HFS associated with anti-VEGF therapy.

The object of the present invention is further solved by providing Vitamin K1 for use in the manufacture of a pharmaceutical formulation for treating or preventing skin reactions associated with anti-VEGF-therapy, such as skin rash or HFS, wherein the pharmaceutical formulation is formulated for topical.

The object of the present invention is also solved by a pharmaceutical formulation comprising vitamin K1 for use in a method for treating or preventing a skin reaction associated with anti-VEGF-therapy, such as skin rash or Hand Foot Syndrome (HFS).

Furthermore, the object of the present invention is also solved by a method of treating and/or preventing a skin reaction associated with anti-VEGF-therapy, such as skin rash or Hand Foot Syndrome (HFS), wherein vitamin K1 is applied to a patient.

The object of the present invention is furthermore solved by the use a vitamin K1 for the manufacture of a medicament for the treatment of a skin reaction associated with anti-VEGF-therapy, such as skin rash or Hand Foot Syndrome (HFS).

### Detailed description of the invention

According to the present invention, Vitamin K1 refers to 2-methyl-3-phytyl-1, 4-naphthoquinone, which is represented by molecular formula as C₃₁H₄₆O₂. It is also sometimes referred to as phytomenadione, phylloquinone or phytonadione. The term vitamin K1, as used herein, is also meant to encompass pharmacologically acceptable salts of 2-methyl-3-phytyl-1,4-naphthohydroquinone, e.g. the sodium or potassium salts of 2-methyl-3-phytyl-1,4-naphthohydroquinone phosphate, e.g. the disodium salt of 2-methyl-3-phytyl-1,4-naphthohydroquinone diphosphate (dihydrovitamin K₁ diphosphate).

In one aspect, the present invention relates to Vitamin K1 having the general formula as listed below:

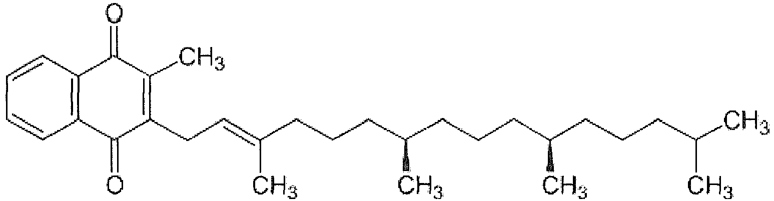

In one embodiment, Vitamin K1 is for use in a method for treating or preventing skin reactions associated with anti-VEGF therapy such as skin rash and Hand Foot Syndrome.

The objects of the present invention are solved by Vitamin K1 for use in a method for treating or preventing a skin reaction associated with anti-VEGF therapy such as skin rash or Hand Foot Syndrome (HFS).

In one embodiment the anti-VEGF therapy is a treatment with a VEGF-inhibitor or a combination of two or more VEGF-inhibitors.

In one embodiment the VEGF-inhibitor is sunitinib, sorafenib, bevacizumab, vatalanib, pegaptanib, or a combination of any of the foregoing.

In one embodiment the Vitamin K1 is formulated for topical administration.

In one embodiment the Vitamin K1 is applied to the skin in a formulation at a concentration of vitamin K1 in said formulation in the range of from 0.05% (w/w) to 4.5% (w/w).

In one embodiment said concentration of vitamin K1 in said formulation is in the range of from 0.1 % (w/w) to 2.0% (w/w), preferably 0.1 % (w/w) to 1% (w/w).

In one embodiment Vitamin K1 is formulated as a sustained release formulation.

In one embodiment said sustained release formulation is in the form of liposomes or vitamin K1-polymer conjugates comprising Vitamin K1, or in the form of a patch comprising a matrix containing vitamin K1, or in the form of micro-capsules wherein said vitamin K1 is encapsulated.

In one embodiment the Vitamin K1 is formulated as an immediate-release formulation.

In one embodiment the Vitamin K1 is formulated in a formulation selected from the group comprising a cream, an ointment, a gel, a salve, an elixir, a lotion, a solution, a patch, an emulsion, a foam, or a spray.

In one embodiment the Vitamin K1 is applied to the face or trunk or any part of body skin including scalp, palms, soles or nails, affected with a skin reaction associated with anti-VEGF therapy such as skin rash or Hand Foot Syndrome.

In one embodiment said Vitamin K1 is administered to a patient, in particular a human patient.

In one embodiment said administration occurs by topical application.

In one embodiment said administration by topical application occurs by applying said vitamin K1 using a device such as a paddle, a brush, a sprayer, a sticking plaster, a patch, a bandage, a dressing, an adhesive bandage, or a pack of powder or one or several devices together.

The objects of the present invention are also solved by a pharmaceutical formulation comprising vitamin K1 for use in a method for treating or preventing a skin reaction associated with anti-VEGF therapy, such as skin rash or Hand Foot Syndrome (HFS).

The objects of the present invention are also solved by a method of treatment or prevention of a skin reaction associated with anti-VEGF-therapy, such as skin rash or Hand Foot Syndrome (HFS), said method comprising the administration of vitamin K1 or of a formulation comprising vitamin K1 to a patient, wherein preferably said patient, said skin reaction, said vitamin K1 and formulation thereof are as defined above or as defined hereafter.

The term "anti-VEGF-therapy", as used herein, is meant to refer to any therapy (involving the administration of one or several drugs) which therapy is aimed at inhibition of the vascular endothelial growth factor (VEGF) or the vascular endothelial growth factor receptor (VEGF-receptor). In one embodiment such anti-VEGF-therapy is a therapy for the treatment of a cancerous disease.

The term "skin rash", as used herein, is meant to refer to a reddening of the skin and/or a swelling of the skin, possibly associated with heat sensation and/or a roughening of the skin. A skin rash may manifest itself in the colour, appearance or texture of the skin. It may be localised in one part of the body or it may effect the entire body surface. A skin rash may cause the skin change colour, itch, become warm, bumpy, chapped, dry, cracked or blistered, swell and may be painful.

The term "Hand Foot Syndrome (HFS)", as used herein, is meant to refer to a side effect of chemotherapeutical treatment or of biologic therapy drugs used to treat certain cancers. Hand Foot Syndrome manifests itself in redness, tenderness and possible peeling and/or skin damage of the palms and soles. More specifically, the term, as used herein, is meant to refer to such symptoms associated with anti-VEGF-therapy.

The term "associated with", as used herein, is meant to refer to a temporal link between events. For example a skin reaction which is associated with anti-VEGF-therapy is a skin reaction which occurs during or after such anti-VEGF-therapy. The term as such does not imply a causal relation, but may also include such causality.

An "anti-VEGF-inhibitor", as used herein, is meant to refer to any drug that inhibits either vascular endothelial growth factor (VEGF) or the vascular endothelial growth factor-receptor (VEGF-receptor). Examples of VEGF-inhibitors are bevacizumab and pegaptanib, whereas examples for VEGF-receptor-inhibitors are sunitinib and sorafenib.

The term "to treat", as used herein, is meant to refer to a medical activity aimed at alleviating, reducing, minimizing, halting or even healing a disease or disease state.

The term "to prevent", as used herein, is meant to refer to any medical activity aimed at averting, hindering, impeding, inhibiting, obstructing, precluding, prohibiting, anticipating or avoiding the onset of a disease or disease state in particular of a skin rash or Hand Foot Syndrome, more specifically in the context of anti-VEGF-therapy.

According to the present invention, vitamin K1 is formulated in a medicament which medicament comprises said vitamin K1 and, optionally, one or more pharmaceutically acceptable ingredients as carriers, excipients, adjuvants, buffers, diluents and/or other customary pharmaceutical auxiliaries. Optionally, the formulation may also comprise one or more therapeutic and/or prophylactic pharmaceutical ingredients. The carrier(s)/excipient(s), buffer(s), adjuvant(s), diluent(s) and auxiliary (auxiliaries) must be "acceptable" in the sense of being compatible with the other ingredients of the formulation and must not be harmful to the recipient thereof.

A medicament/formulation of the invention is a formulation that is, in particular, suitable for topical administration. The formulation may be a sustained-release formulation or it may be an immediate-release formulation. A person skilled in the art knows how to formulate an appropriate sustained-released formulation. This may for example be in the form of liposomes or microcapsules enclosing vitamin K1, or in the form of a suitable matrix or film containing vitamin K1. In one embodiment such sustained-release system includes a semipermiable matrix, wherein said matrix contains vitamin K1. Appropriate formulations and ways of manufacturing them are well-known to a person skilled in the art and are, for example disclosed in "Arzneiformenlehre, Paul Heinz List, Ein Lehrbuch fur Pharmazeuten, Wissenschaftliche Verlagsgesellschaft Stuttgart, 4. Auflage, 1985", or "The theory and practice of industrial pharmacy" by Lachman et al., Varghese Publishing House, 1987", or "Modern Pharmaceutics", edited by James Swarbrick, 2. Edition".

In a preferred embodiment, vitamin K1 is prepared and/or formulated in the form of an ointment, a gel, a plaster, an emulsion, a lotion, a foam, a cream, a cream of a mixed phase or amphiphilic emulsion of a mixed phase or amphiphilic emulsion system (oil/water, water/oil-mixed phase), a paste, a solution, a patch, an elixir, or a spray. Ointments and creams may, for example be formulated with an aqueous or oily base with the edition of suitable thickening and/or gelling agents, depending on the desired qualities. Lotions may be formulated with aqueous or oily base and will, in general, also contain one or more emulsifying agents, stabilising agents, dispersing agents, suspending agents, thickening agents, or coloring agents.

In one embodiment, a formulation according to the present invention containing vitamin K1 comprises such vitamin K1 in a concentration in the range of from 0.05% (w/w) to 4.5% (w/w), preferably in the range from 0.1 % (w/w) to 2.0% (w/w), more preferably 0.1 % (w/w) to 1%(w/w).

Further details on techniques for formulation and administration may for example be found in Remington: The science and practice of pharmacy", Lippincott Williams and Wilkins.

In one embodiment, a formulation according to the present invention comprising vitamin K1 may additionally comprise a further pharmaceutical ingredient suitable for alleviating or treating skin diseases, including but not limited to antifungal agents, moisturising agents, cortical steroids, antibacterials, retinoids, antibiotics, immunosuppressants and antiviral agents. Additionally and/or alternatively, such formulation may also include other common skin scare components, such as aloe vera, allantoin and/or urea. In a preferred embodiment, the formulation according to the present invention contains 0.05% (w/w) - 1.5% (w/w) vitamin K1, 1.5% (w/w) - 2.5% (w/w) urea, and 1.5% (w/w) - 2.5% (w/w) allantoin. In a more preferred embodiment, a formulation according to the present invention contains 0.1% (w/w) vitamin K1, 2% (w/w) urea, and 1.7% (w/w) allantoin.

A commercially available example that is useful in the practice of the present invention is "reconval^{®}K1", available from Pharmadab, Ljubljana, Slowenia.

The term "patient" means any recipient of health care services, who suffers or is likely to suffer or is suspected of suffering from the side effects of receiving anti-VEGF therapy. Patients receiving anti-VEGF therapy include patients receiving treatment with VEGF inhibitors, such as sunitinib and / or sorafenib and / or any other VEGF inhibitors having side effects on dermatological conditions.

The term "Adverse Event" or "adverse reactions" as used herein is as any unfavourable or unintended symptom, sign, or disease (including an abnormal laboratory finding), temporally or permanently associated with the use of a medical treatment or procedure that may or may not be considered related to the medical treatment or procedure. This definition is derived from the Common Toxicity Criteria Manual Version 2.0.

The term "skin reaction" means any reaction on the skin that is caused by or associated with anti-VEGF therapy, which can be but is not limited to skin rash, HFS, dry skin, flushing, hyperpigmentation, nail changes, photosensitivity, radiation recall, etc. In the present invention, the term "skin reaction" refers to adverse events on the skin caused by or associated with anti-VEGF therapy.

As used herein, the evaluation of HFS in the below examples and embodiments was made according to Common Toxicity Criteria (CTC) Version 2.0 of the Cancer Therapy Evaluation Program from National Cancer Institute (http://www.cancer.gov/). CTC was first developed in 1982 for use in adverse drug experience and has been used widely for collecting treatment-related adverse event data to facilitate the evaluation of new cancer therapies, treatment modalities and supportive measures. The 2.0 Version of CTC was published in April 30, 1999. According to CTC (page 8), the graduation of HFS can be classified to 3 grades, which is introduced in Table 1.

| Adverse Event | Grade | | | |
|---|---|---|---|---|
| | 0 | 1 | 2 | 3 |
| Hand-foot skin reaction | none | Skin changes or dermatitis without pain (e.g., erythema, peeling) | Skin changes with pain, not interfering with function | Skin changes with pain, interfering with function |

In the following, reference is made to the examples which are given to illustrate not to limit the present invention.

### Examples

### 1. Subjects

Subjects to this experiment were two patients receiving anti-VEGF therapies. Patient 1 (Example 1) received a VEGF inhibitor as treatment for advanced renal cell carcinoma (sorafenib). Patient 2 (Example 2) received a VEGF inhibitor as treatment for ovarian cancer (also sorafenib).

### 2. Information about anti-VEGF treatment

The recommended treatment of anti-VEGF for cell carcinoma is once every two weeks. The recommended treatment of anti-VEGF for ovarian cancer is 6 cycles repeated every 3 weeks. After ending a cycle of specific treatment, the patient has time for recovery. Treatment with cream K1 is repeated for each cycle in accordance with the primary medicine/drug.

### 3. Pharmaceucial preparation

Reconval® K1 as an example of an embodiment of the present invention is a topical protective cream for restoring the physiological conditions of the skin damages by external and chemical factors. This cream contains 2% urea, 1.7% allantoin and 0.1% Vitamin K1.

### Example 1

The patient developed Hand Foot Syndrome with grade 3 toxicity within 30 days of anti-VEGF therapy. Graduation was classified according to the National Cancer Institute-Common Toxicity Criteria v2.0. Grade 3 toxicity represents skin changes with pain, interfering with function, Patient developed clinical presentation of Hand Foot Syndrome reaction of both palms (left and right palm). Patient started to apply cream with 0.1% vitamin K1 (Reconval^{®}K1) on right palm twice per day, the left palm was treated with a skin moisturizer only. Control was performed after 14 days.

After the treatment with the respective creams, the patient's right palm was down-staged for 2 grades and patient's left palm had the same grade 3.

### Example 2

Patient 2 received VEGF inhibitor for ovarian cancer. A skin rash developed after the first application of the VEGF inhibitor. The skin rash affected face and upper thorax, grade 2. Graduation was classified according to the National Cancer institute-Common Toxicity Criteria v2.0. Grade 2 toxicity represents skin changes with pain, not interfering with function.

The patient applied cream with vitamin K1 (Reconval^{®}K1) only on the face twice per day. Time to improvement was 10 days. After such treatment with cream, the skin rash on the face, but not on the upper thorax, was down staged by 1 grade.

It is understood that the examples and embodiments described herein are for illustrative purposes only and that the scope of of the invention will be defined by the appended claims.

## Claims

1. Vitamin K1 or a pharmaceutically acceptable salt of 2-methyl-3-phytyl-1,4-naphthohydroquinone for use in a method for treating or preventing a skin reaction associated with anti-VEGF therapy such as skin rash or Hand Foot Syndrome (HFS).

2. Vitamin K1 or a pharmaceutically acceptable salt of 2-methyl-3-phytyl-1,4-naphthohydroquinone for use according to claim 1, wherein the anti-VEGF therapy is a treatment with a VEGF-inhibitor or a combination of two or more VEGF-inhibitors.

3. Vitamin K1 or a pharmaceutically acceptable salt of 2-methyl-3-phytyl-1,4-naphthohydroquinone for use according to claim 2, wherein the VEGF-inhibitor is sunitinib, sorafenib, bevacizumab, vatalanib, pegaptanib, or a combination of any of the foregoing.

4. Vitamin K1 or a pharmaceutically acceptable salt of 2-methyl-3-phytyl-1,4-naphthohydroquinone for use according to claim 1, wherein the Vitamin K1 is formulated for topical administration.

5. Vitamin K1 or a pharmaceutically acceptable salt of 2-methyl-3-phytyl-1,4-naphthohydroquinone for use according to claim 4, wherein the Vitamin K1 is applied to the skin in a formulation at a concentration of vitamin K1 in said formulation in the range of from 0.05% (w/w) to 4.5% (w/w).

6. Vitamin K1 or a pharmaceutically acceptable salt of 2-methyl-3-phytyl-1,4-naphthohydroquinone for use according to claim 5, wherein said concentration of vitamin K1 in said formulation is in the range of from 0.1% (w/w) to 2.0% (w/w), preferably 0.1% (w/w) to 1% (w/w).

7. Vitamin K1 or a pharmaceutically acceptable salt of 2-methyl-3-phytyl-1,4-naphthohydroquinone for use according to any of the foregoing claims, wherein Vitamin K1 is formulated as a sustained release formulation.

8. Vitamin K1 or a pharmaceutically acceptable salt of 2-methyl-3-phytyl-1,4-naphthohydroquinone for use according to claim 7, wherein said sustained release formulation is in the form of liposomes or vitamin K1-polymer conjugates comprising Vitamin K1, or in the form of a patch comprising a matrix containing vitamin K1, or in the form of micro-capsules wherein said vitamin K1 is encapsulated.

9. Vitamin K1 or a pharmaceutically acceptable salt of 2-methyl-3-phytyl-1,4-naphthohydroquinone for use according to any of claims 1-6, wherein the Vitamin K1 is formulated as an immediate-release formulation.

10. Vitamin K1 or a pharmaceutically acceptable salt of 2-methyl-3-phytyl-1,4-naphthohydroquinone for use according to any of the foregoing claims, wherein the Vitamin K1 is formulated in a formulation selected from the group comprising a cream, an ointment, a gel, a salve, an elixir, a lotion, a solution, a patch, an emulsion, a foam, or a spray.

11. Vitamin K1 or a pharmaceutically acceptable salt of 2-methyl-3-phytyl-1,4-naphthohydroquinone for use according to claim 10, wherein the Vitamin K1 is applied to the face or trunk or any part of body skin including scalp, palms, soles or nails, affected with a skin reaction associated with anti-VEGF therapy such as skin rash or Hand Foot Syndrome.

12. Vitamin K1 or a pharmaceutically acceptable salt of 2-methyl-3-phytyl-1,4-naphthohydroquinone for use according to claim 11, wherein said Vitamin K1 is administered to a patient, in particular a human patient.

13. Vitamin K1 or a pharmaceutically acceptable salt of 2-methyl-3-phytyl-1,4-naphthohydroquinone for use according to claim 12, wherein said administration occurs by topical application.

14. Vitamin K1 or a pharmaceutically acceptable salt of 2-methyl-3-phytyl-1,4-naphthohydroquinone for use according to claim 13, wherein said administration by topical application occurs by applying said vitamin K1 using a device such as a paddle, a brush, a sprayer, a sticking plaster, a patch, a bandage, a dressing, an adhesive bandage, or a pack of powder or one or several devices together.

15. Pharmaceutical formulation comprising vitamin K1 or a pharmaceutically acceptable salt of 2-methyl-3-phytyl-1,4-naphthohydroquinone for use in a method for treating or preventing a skin reaction associated with anti-VEGF therapy, such as skin rash or Hand Foot Syndrome (HFS).

## Patentansprüche

1. Vitamin K1 oder ein pharmazeutisch akzeptables Salz von 2-Methyl-3-phytyl-1,4-naphthohydrochinon zur Verwendung in einem Verfahren zur Behandlung oder Verhinderung einer Hautreaktion, die mit einer anti-VEGF-Therapie verbunden ist, wie etwa ein Hautausschlag oder Hand-Fuß-Syndrom (HFS).

2. Vitamin K1 oder ein pharmazeutisch akzeptables Salz von 2-Methyl-3-phytyl-1,4-naphthohydrochinon zur Verwendung nach Anspruch 1, wobei die anti-VEGF-Therapie eine Behandlung mit einem VEGF-Inhibitor oder einer Kombination von zwei oder mehreren VEGF-Inhibitoren ist.

3. Vitamin K1 oder ein pharmazeutisch akzeptables Salz von 2-Methyl-3-phytyl-1,4-naphthohydrochinon zur Verwendung nach Anspruch 2, wobei der VEGF-Inhibitor Sunitinib, Sorafenib, Bevacizumab, Vatalanib, Pegaptanib oder eine Kombination der vorhergehenden ist.

4. Vitamin K1 oder ein pharmazeutisch akzeptables Salz von 2-Methyl-3-phytyl-1,4-naphthohydrochinon zur Verwendung nach Anspruch 1, wobei das Vitamin K1 zur topischen Verabreichung formuliert ist.

5. Vitamin K1 oder ein pharmazeutisch akzeptables Salz von 2-Methyl-3-phytyl-1,4-naphthohydrochinon zur Verwendung nach Anspruch 4, wobei das Vitamin K1 auf die Haut in einer Formulierung mit einer Konzentration von Vitamin K1 in der Formulierung im Bereich von 0,05% (Gew./Gew.) bis 4,5% (Gew./Gew.) aufgetragen wird.

6. Vitamin K1 oder ein pharmazeutisch akzeptables Salz von 2-Methyl-3-phytyl-1,4-naphthohydrochinon zur Verwendung nach Anspruch 5, wobei die Konzentration von Vitamin K1 in der Formulierung im Bereich von 0,1% (Gew./Gew.) bis 2,0% (Gew./Gew.), bevorzugt 0,1% (Gew./Gew.) bis 1,0% (Gew./Gew.) ist.

7. Vitamin K1 oder ein pharmazeutisch akzeptables Salz von 2-Methyl-3-phytyl-1,4-naphthohydrochinon zur Verwendung nach einem der vorangehenden Ansprüche, wobei Vitamin K1 als eine Formulierung mit verzögerter Freisetzung formuliert ist.

8. Vitamin K1 oder ein pharmazeutisch akzeptables Salz von 2-Methyl-3-phytyl-1,4-naphthohydrochinon zur Verwendung nach Anspruch 7, wobei die Formulierung mit verzögerter Freisetzung in der Form von Liposomen oder Vitamin K1-Polymer-Konjugaten, umfassend Vitamin K1, oder in der Form eines Pflasters, das eine Vitamin K1-enhaltende Matrix umfasst, oder in der Form von Mikrokapseln ist, wobei das Vitamin K1 verkapselt ist.

9. Vitamin K1 oder ein pharmazeutisch akzeptables Salz von 2-Methyl-3-phytyl-1,4-naphthohydrochinon zur Verwendung nach einem der Ansprüche 1-6, wobei das Vitamin K1 als eine Formulierung mit unmittelbarer Freisetzung formuliert ist.

10. Vitamin K1 oder ein pharmazeutisch akzeptables Salz von 2-Methyl-3-phytyl-1,4-naphthohydrochinon zur Verwendung nach einem der vorangehenden Ansprüche, wobei das Vitamin K1 in einer Formulierung formuliert ist, ausgewählt aus der Gruppe, umfassend eine Creme, ein Balsam, ein Gel, eine Salbe, ein Elixier, eine Lotion, eine Lösung, ein Pflaster, eine Emulsion, ein Schaum oder ein Spray.

11. Vitamin K1 oder ein pharmazeutisch akzeptables Salz von 2-Methyl-3-phytyl-1,4-naphthohydrochinon zur Verwendung nach Anspruch 10, wobei das Vitamin K1 auf das Gesicht oder den Rumpf oder einen Teil der Körperhaut aufgetragen wird, einschließlich Kopfhaut, Handflächen, Sohlen oder Nägeln, die von einer Hautreaktion betroffen ist, die mit anti-VEGF-Therapie verbunden ist, wie etwa Hautausschlag oder Hand-Fuß-Syndrom.

12. Vitamin K1 oder ein pharmazeutisch akzeptables Salz von 2-Methyl-3-phytyl-1,4-naphthohydrochinon zur Verwendung nach Anspruch 11, wobei das Vitamin K1 an einen Patienten verabreicht wird, insbesondere einen menschlichen Patienten.

13. Vitamin K1 oder ein pharmazeutisch akzeptables Salz von 2-Methyl-3-phytyl-1,4-naphthohydrochinon zur Verwendung nach Anspruch 12, wobei die Verabreichung mittels topischer Verabreichung erfolgt.

14. Vitamin K1 oder ein pharmazeutisch akzeptables Salz von 2-Methyl-3-phytyl-1,4-naphthohydrochinon zur Verwendung nach Anspruch 13, wobei die Verabreichung mittels topischer Auftragung stattfindet, indem Vitamin K1 unter Verwendung einer Vorrichtung aufgetragen wird, wie einem Paddel, einer Bürste, einem Sprayer, einem klebenden Pflaster, einem Pflaster, einer Bandage, einem Verband, einem Klebeverband oder einer Pulverpackung oder einer oder mehrerer Vorrichtungen zusammen.

15. Pharmazeutische Formulierung, umfassend Vitamin K1 oder ein pharmazeutisch akzeptables Salz von 2-Methyl-3-phytyl-1,4-naphthohydrochinon, zur Verwendung in einem Verfahren zur Behandlung oder Verhinderung einer Hautreaktion, die mit anti-VEGF-Therapie verbunden ist, wie etwa Hautausschlag oder Hand-Fuß-Syndrom (HFS).

## Revendications

1. Vitamine K1 ou un sel pharmaceutiquement acceptable de 2-méthyl-3-phytyl-1,4-naphtohydroquinone pour une utilisation dans un procédé de traitement ou de prévention d'une réaction cutanée associée à un traitement anti-VEGF telle qu'un rash cutané ou le syndrome main-pied (SMP).

2. Vitamine K1 ou un sel pharmaceutiquement acceptable de 2-méthyl-3-phytyl-1,4-naphtohydroquinone pour une utilisation selon la revendication 1, dans lequel le traitement anti-VEGF est un traitement avec un inhibiteur du VEGF ou une combinaison de deux inhibiteurs du VEGF ou plus.

3. Vitamine K1 ou un sel pharmaceutiquement acceptable de 2-méthyl-3-phytyl-1,4-naphtohydroquinone pour une utilisation selon la revendication 2, dans lequel l'inhibiteur du VEGF est le sunitinib, le sorafénib, le bévacizumab, le vatalanib, le pégaptanib, ou une combinaison quelconque de ceux-ci.

4. Vitamine K1 ou un sel pharmaceutiquement acceptable de 2-méthyl-3-phytyl-1,4-naphtohydroquinone pour une utilisation selon la revendication 1, dans lequel la vitamine K1 est formulée pour une administration topique.

5. Vitamine K1 ou un sel pharmaceutiquement acceptable de 2-méthyl-3-phytyl-1,4-naphtohydroquinone pour une utilisation selon la revendication 4, dans lequel la vitamine K1 est appliquée sur la peau dans une formulation à une concentration de vitamine K1 dans ladite formulation située dans la plage de 0,05 % (p/p) à 4,5 % (p/p).

6. Vitamine K1 ou un sel pharmaceutiquement acceptable de 2-méthyl-3-phytyl-1,4-naphtohydroquinone pour une utilisation selon la revendication 5, dans lequel ladite concentration de vitamine K1 dans ladite formulation se situe dans la plage de 0,1 % (p/p) à 2,0 % (p/p), de préférence 0,1 % (p/p) à 1 % (p/p).

7. Vitamine K1 ou un sel pharmaceutiquement acceptable de 2-méthyl-3-phytyl-1,4-naphtohydroquinone pour une utilisation selon l'une quelconque des revendications précédentes, dans lequel la vitamine K1 est formulée sous la forme d'une formulation à libération prolongée.

8. Vitamine K1 ou un sel pharmaceutiquement acceptable de 2-méthyl-3-phytyl-1,4-naphtohydroquinone pour une utilisation selon la revendication 7, dans lequel ladite formulation à libération prolongée est sous la forme de liposomes ou de conjugués de vitamine K1-polymère comprenant de la vitamine K1, ou sous la forme d'un patch comprenant une matrice contenant de la vitamine K1, ou sous la forme de microcapsules dans lesquelles ladite vitamine K1 est encapsulée.

9. Vitamine K1 ou un sel pharmaceutiquement acceptable de 2-méthyl-3-phytyl-1,4-naphtohydroquinone pour une utilisation selon l'une quelconque des revendications 1 à 6, dans lequel la vitamine K1 est formulée sous la forme d'une formulation à libération immédiate.

10. Vitamine K1 ou un sel pharmaceutiquement acceptable de 2-méthyl-3-phytyl-1,4-naphtohydroquinone pour une utilisation selon l'une quelconque des revendications précédentes, dans lequel la vitamine K1 est formulée dans une formulation choisie dans le groupe comprenant une crème, une pommade, un gel, un baume, un élixir, une lotion, une solution, un patch, une émulsion, une mousse, ou une pulvérisation.

11. Vitamine K1 ou un sel pharmaceutiquement acceptable de 2-méthyl-3-phytyl-1,4-naphtohydroquinone pour une utilisation selon la revendication 10, dans lequel la vitamine K1 est appliquée sur le visage ou le tronc ou une partie quelconque de la peau du corps y compris le cuir chevelu, les paumes des mains, les plantes des pieds ou les ongles, affectée par une réaction cutanée associée à un traitement anti-VEGF telle qu'un rash cutané ou le syndrome main-pied.

12. Vitamine K1 ou un sel pharmaceutiquement acceptable de 2-méthyl-3-phytyl-1,4-naphtohydroquinone pour une utilisation selon la revendication 11, dans lequel ladite vitamine K1 est administrée à un patient, en particulier un patient humain.

13. Vitamine K1 ou un sel pharmaceutiquement acceptable de 2-méthyl-3-phytyl-1,4-naphtohydroquinone pour une utilisation selon la revendication 12, dans lequel ladite administration survient par application topique.

14. Vitamine K1 ou un sel pharmaceutiquement acceptable de 2-méthyl-3-phytyl-1,4-naphtohydroquinone pour une utilisation selon la revendication 13, dans lequel ladite administration par application topique survient par application de ladite vitamine K1 en utilisant un dispositif tel qu'une spatule, une brosse, un pulvérisateur, un emplâtre adhésif, un patch, un bandage, un pansement, un bandage adhésif, ou un paquet de poudre ou un ou plusieurs dispositifs ensemble.

15. Formulation pharmaceutique comprenant de la vitamine K1 ou un sel pharmaceutiquement acceptable de 2-méthyl-3-phytyl-1,4-naphtohydroquinone pour une utilisation dans un procédé de traitement ou de prévention d'une réaction cutanée associée à un traitement anti-VKGF, telle qu'un rash cutané ou le syndrome main-pied (SMP).
